# EUROPEAN PATENT APPLICATION

(11) **EP 1 300 128 A1**
(43) Date of publication of application: **09.04.2003**
(21) Application number: 01947948.4
(22) Date of filing: 10.07.2001
(51) Int. Cl.: A61J 1/00

(54) **BLOOD PROCESSING FILTER**

(30) Priority: 10.07.2000 JP 2000208736; 10.07.2000 JP 2000208737
(71) Applicant: ASAHI MEDICAL Co., Ltd., Tokyo 101-8482 (JP)
(72) Inventor: TSUJI, Michihiro, Oita-shi, Oita 870-0304 (JP); OKA, Shin-ichiroh, Oita-shi, Oita 870-0163 (JP)
(74) Representative: von Kreisler, Alek, Dipl.-Chem.
(86) International application number: JP0105965
(87) International publication number: WO02003909

(57) **Abstract**

The invention relates to a blood processing filter comprising a flexible container having an inlet port and an outlet port for the blood and a sheet-like filter element for removing undesirable components from blood, wherein the filter element separates the inlet port from the outlet port for the blood, a blood processing filter characterized by comprising: a first seal zone formed by integrating the entire circumference in the vicinity of the periphery of the filter element with the flexible container; a second seal zone formed by integrating the inlet port side flexible container with the outlet port side flexible container over the entire outer circumference of the first seal zone; and an unsealed zone with a width of 1-30 mm between the first seal zone and the second seal zone.

## Description

### TECHNICAL FIELD

The present invention relates to a blood processing filter for removing undesirable components such as aggregates and leukocytes from the blood. The present invention particularly relates to a precise and disposable blood processing filter to be used for removing micro aggregates and leukocytes, that cause side effects, from whole blood preparations, erythrocyte preparations, thrombocyte preparations, blood plasma preparations and the like for use in blood transfusion. Especially, the blood processing filter of the present invention is most suitable for being centrifuged together with blood bags and the like in the centrifugation operation carried out with an objective of separating blood components.

### BACKGROUND ART

The whole blood collected from a donor is used for transfusion, as is, only in rare cases, but is commonly. separated into components, such as a erythrocyte preparation, thrombocyte preparation, blood plasma preparation, and the like. Each component is then stored and is used for transfusion thereafter. Since micro aggregates and leukocytes contained in these blood preparations cause various side effects after transfusion, there have been increasing occasions when these undesirable components are removed before blood transfusion. The need of removing the leukocytes has been widely recognized in recent years, and some European countries legislate the blood preparations to use for transfusion after applying an treatment for removing leukocytes. The most common method for removing the leukocytes from the blood preparation is by processing the blood preparation using a leukocyte removing filter. Conventionally, blood preparation has been processed using the leukocyte removing filter in many cases at the bedside when blood transfusion is performed, it is now common to process the blood before storage at the blood center for assuring quality control of the blood preparations after removing the leukocytes, and for improving efficiency of the leukocyte removing treatment. A blood collection-separation set, typically composed of two to four flexible bags, tubes connecting these bags, an anticoagulant, an erythrocyte preservation solution, a blood collecting needle, and the like, has been used for collecting blood from a donor, separating the blood into several blood components, and storing the blood components. A system in which the leukocyte removing filter is integrated/incorporated into the blood collection-separation set has been widely used as a system that can be favorably used for removing of the leukocytes before storage. Such a system is called a "closed system" or an "integrated system" and the like. Such a system is disclosed, for example, in Japanese Patent Application Laid-open Publication No. 01-320064 and WO 92/20428.

Conventionally, a filter made from non-woven fabric or porous filter elements packed in a hard container such as polycarbonate has been widely used as a leukocyte removing filter. However, it has been difficult to use a vapor sterilization method, that is widely used for sterilization of blood collection-separation sets, since the container does not have gas permeability.

In a closed system, leukocytes are first removed from the whole blood preparation after collecting the blood. Then, after the leukocyte removing filter is separated, the leukocyte-free blood is centrifuged for separation into various components. Then, after the leukocyte removing filter is separated, the leukocyte-free blood is centrifuged for separation into various components. In another type of closed system, the whole blood is first centrifuged to be divided into various components, and then the leukocytes are removed. In the latter system, the leukocyte removing filter is also centrifuged together with the blood collection-separation set. In this instance, a hard container may damage bags and tubes, or the container itself may not withstand the stress and may collapse during centrifugation.

To solve this problem, flexible leukocyte removing filters, in which the container is made of the same or a similar material having superior flexibility and high vapor permeability as used for the bags of the blood collection-separation set, have been developed (EP 0526678 and Japanese Patent Application Laid-open Publication No. 11-216179).

However, these leukocyte removing filters have a problem of a complicated manufacturing process, since a sheet of flexible frame must be welded to a housing material after welding the filter element to the flexible frame. It has also been a problem that a large portion of the starting material is wasted since an effective filtration part is produced by punching the sheet inside the frame.

Flexible leukocyte removing filters not using a sheet of the frame are disclosed in Japanese Patent Application Laid-open Publication No. 07-267871 and WO 95/17236. These filters have some risk, however, because the outermost circumference periphery of the filter element are welded and the welded part has become a plate of hard plastic. Similar to conventional filters made of a hard plastic container, the hard plastic may damage bags and tubes. In addition, the welded part has a risk of being broken due to stress during centrifugation.

In particular, the former filter having the outermost peripheries of the filter element welded with the container material cannot avoid the risk of exposing the medical workers to the danger of infection or cannot prevent blood preparations from being contaminated with miscellaneous bacteria, when leaking resulting from breakage of the welded parts due to operational mistakes, rough handling, stress of centrifugal operation, or the like during filtration. On the other hand, the latter leukocyte removing filter, which also has the outermost peripheries of the filter element welded with the container material, is designed to reduce the risk of leaking by covering the peripheries of the filter element with the container material. However, the filter has a structure precluding detection of potential cracks which allow blood to pass through in the welded parts of the filter element. For this reason, even if no leakage to outside takes place, blood may bypass the proper route of the filter element and pass through short passages such as cracks and inappropriately welded parts, resulting in a risk of decreasing the leukocyte removing function. This type of filter cannot thus detect such a risk.

### DISCLOSURE OF THE INVENTION

A first object of the present invention is to provide a blood processing filter that makes it possible to manufacture a flexible blood processing filter without using a sheet-like flexible frame and thus without a complicated manufacturing process or increase of loss of the starting materials. A second object of the present invention is to provide a blood processing filter that can protect the medical workers from the risks of exposure to infections or prevent contamination of the blood preparations with bacteria, even when the seal portions between the filter element and the container are broken to cause leakage by operational mistakes or rough handling during filtration, and by stress during centrifugation. An another object of the present invention is to provide a blood processing filter having a construction which, in the case where the blood by-passes through cracks or insufficiently welded portions without passing through the filter element as a proper passage for the blood, resulting in decreasing the leukocyte removing function of the filter element, can detect such risks by inspection during the manufacturing process of the filter.

As a result of extensive studies to achieve the above objects, the inventors of the present invention have found that the above objects can be surprisingly achieved at the same time by integrating the flexible container with the filter element in a first seal zone, forming a second seal zone integrated the inlet port side flexible container with the outlet port side flexible container outside the first seal zone, and providing an unsealed zone between them.

As a result of a further study with an objective of obtaining a blood processing filter with welded parts not easily broken, the inventors have found that all of the above objects can be achieved by integrating the flexible container with the filter element in the first seal zone, forming the second seal zone to integrate the flexible container outside the first seal zone, and providing an unsealed zone between them, and further providing a peripheral end of the filter element with a width of 2-25 mm over the entire circumference of the unsealed zone. These findings have led to the completion of the present invention.

Specifically, the present invention provides a blood processing filter comprising a flexible container having an inlet port and an outlet port for the blood and a filter element for removing undesirable components from blood, the filter element partitioning the inlet port from the outlet port for the blood, comprising: a first seal zone formed by integrating the entire circumference in the vicinity of the periphery of the filter element with the flexible container; a second seal zone formed by integrating the inlet port side flexible container with the outlet port side flexible container over the entire outer circumference of the first seal zone; and an unsealed zone with a width of 1-30 mm between the first seal zone and the second seal zone.

In another aspect, the present invention provides a blood processing filter comprising a flexible container having an inlet port and an outlet port for the blood, and a filter element for removing undesirable components from the blood, wherein the inlet port and outlet port for the blood are partitioned by the filter element, comprising: a first seal zone formed by integrating the entire circumference in the vicinity of the peripheral end of the filter element with the flexible container; a second seal zone formed by integrating the inlet port side flexible container with the outlet port side flexible container over the entire outer circumference of the first seal zone; an unsealed zone provided between the first seal zone and the second seal zone; and a peripheral end of the filter element with a width of 2-25 mm over the entire circumference of the filter element within the unsealed zone.

The peripheral end of the filter element existing within the unsealed zone is hereinafter referred to as "protruding filter material" from time to time.

### BRIEF DESCRIPTION OF THE DRAWINGS

Figure 1 is a schematic sectional view of one embodiment of the blood processing filter of the present invention.
Figure 2 shows one embodiment of the process for manufacturing the blood processing filter of the present invention.
Figure 3 shows another embodiment of the process for manufacturing the blood processing filter of the present invention.

### BEST MODE FOR CARRYING OUT THE INVENTION

The present invention will be described in more detail below.

The overall shape of the blood processing filter of the present invention may be rectangular, lozenge-shaped, disk-like, oval, or the like. A rectangular or lozenge-shaped filter is preferable for decreasing loss of materials when manufacturing the filters. In the present invention, a square is classed as a rectangle.

The flexible container used in the present invention is preferably formed from a flexible sheet-like or a cylindrically formed object of a synthetic resin, preferably a thermoplastic resin. The flexible container may be formed by injection molding or the like as an integrally molded body with an inlet port and outlet port for the blood. Alternatively, holes or slits may be formed on a sheet or cylinder of film manufactured by extrusion molding, to which an separately molded parts for inlet port and outlet port are liquid-tightly and communicatingly connected by a known method, such as a method of using an adhesive, heat sealing, high frequency welding, or the like. The latter method is more preferable because the container is deformed only with difficulty during vapor sterilization. The material of the inlet and outlet parts may be the same as or different from the material of the molded film. Although the material is not particularly restricted so far as the inlet port and outlet port are capable of being joined to the molded film with no gaps and of being liquid-tight, in addition to causing no trouble in handling, the materials preferably have similar thermal and electrical properties to the molded film, because heat sealing and high frequency molding methods are advantageously used for mass production. Suitable joining is possible by the high frequency welding method when materials having a relatively high dielectric constant such as soft vinyl chloride are welded to one another, while the material having a relatively low dielectric constant and a low melting point such as polyolefin can be favorably joined by heat sealing.

The material for the flexible container preferably has thermal and electrical properties similar to the material for the filter element. As examples of such a suitable material, thermoplastic elastomers such as soft polyvinyl chloride, polyurethane, an ethylene-vinyl acetate copolymer, polyolefin such as polyethylene and polypropylene, hydrogenated styrene-butadiene-styrene copolymer, styrene-isoprene-styrene copolymer or the hydrogenated product thereof, and mixtures of the thermoplastic elastomer and a softening agent such as polyolefin and ethylene-ethyl acrylate, and the like can be given. Of these, preferable materials are thermoplastic elastomers such as soft polyvinyl chloride, polyurethane, ethylene-vinyl acetate copolymer, polyolefin, and mixtures containing these thermoplastic elastomers as a major component, with particularly preferable materials being soft polyvinyl chloride and polyolefin.

While any material is suitable for the sheet of the filter element in the present invention so far as it can remove undesirable components from the blood, the materials preferably include such a filter element that is able to remove leukocytes. More preferably, the filter element of the present invention includes the first filter element for removing aggregates from the blood at the inlet side, the second filter element for removing the leukocytes, and the third filter element arranged for preventing adhesion of the element for removing the leukocytes to the outlet port side container.

Filter media known in the art such as a fibrous and porous medium such as a non-woven fabric and a porous medium having continuous pores of a three-dimensional network may be used as the filter element in the present invention. The materials for such fiber media include polypropylene, polyethylene, styrene-isobutylene-styrene copolymer, polyurethane, polyester, and the like.

A combination of filter elements having different fiber diameters and pore sizes is usually used. In the case of the filter element comprising the first to third filter elements, a filter material having a fiber diameter of several to several tens of microns is arranged as the first filter element for removing aggregates, a filter material having a fiber diameter of 0.3-3.0 µm is then arranged as the second filter element for removing leukocytes, and a filter material having a fiber diameter of several to several tens of microns is laminated as the third filter element between the second filter element and the outlet port side container for preventing the outlet side container from adhering to the second filter element.

Each of the first, second, and third filter elements may be formed from two or more different filter elements. In this instance, these filter elements are preferably arranged so that the fiber diameter increases stepwise or continuously from the portion of the second filter element having the smallest fiber diameter toward the inlet and the outlet.

In the same manner, when porous materials having a three-dimensional network of continuous fine pores are used, the filter elements are preferably arranged so that the pore size increases stepwise or continuously from the portion of the second filter element with the smallest pore size toward the inlet and the outlet.

A method such as internal welding by high frequency welding or by supersonic wave welding, external welding by heat sealing, adhesion using a potting agent, or the like can be used for forming the first seal zone, specifically, for joining the flexible container with the vicinity of the periphery of the filter element. The high frequency welding method is preferably used when both the flexible container and the filter element are made from materials with a comparatively high dielectric constant, and heat sealing is preferably used when either material has a low dielectric constant or both materials have a low melting point.

The first seal zone may be formed either by a two-step welding method by which, after the vicinity of the periphery of the filter element is once welded, the welded portion is further welded to the flexible container, or by a one step welding method by which the filter element and the flexible container are simultaneously welded. The one step welding method is more preferable for simplifying the manufacturing process.

Although the first seal zone is not necessarily formed by joining the entire filter element to the flexible container, at least the filter element for removing the leukocytes must be welded to the flexible container, when the filter element includes the filter element for removing the leukocytes in addition to a laminated layer having different functions. This is because if the filter element for removing the leukocytes is not integrated with the flexible container, the leukocyte removing function deteriorates due to bypassing.

Although the width of the first seal zone is not particularly restricted, it is preferably within a range of 1-7 mm, more preferably 2-5 mm, in view of reliability of the seal and easy handling of the filter. If less than 1 mm, the joined part becomes like a line, which has a risk of failing to exhibit sufficient sealing performance when subjected to high-pressure vapor sterilization or roughly handled. If the width is larger than 7 mm, the characteristics as the flexible container are partly lost because the width of the seal zone portion that tends to be hardened by high frequency welding, heat sealing, or impregnation of the potting agent becomes too large. This unfavorably causes the filter element to become fragile against bending stress or deformation occurring during centrifugation, precluding the protruding filter material from sufficiently exhibiting its protective effect.

The first seal zone may be formed either in the outermost periphery of the filter element or in a portion slightly inside the outermost periphery. The latter case is more preferable, particularly when the first seal zone is formed by high frequency welding or by heat sealing. Specifically, it is desirable to ensure superior process stability that the first seal zone is formed in an inner position than a point of 2-25 mm inside from the peripheral end of the filter element so that about a several mm margin may be left unsealed outside the first seal zone.

The unsealed filter element formed within the unsealed zone must be present over the entire circumference with a width of 2-25 mm. When the blood processing filter is centrifuged together with the blood collection-separation set, the protruding filter material functions as a cushion, protecting the blood bags and circuits of the blood collection-separation set from being harmed and, at the same time, reducing the risk of the blood processing filter being damaged by the centrifuge operation.

One typical example of the damage to the blood processing filter due to centrifuge operation will be described. There are various types of centrifuge cups and the manner of arranging the blood collection-separation set and the blood processing filter in a centrifuge cup varies according to the type of the centrifuge cup. Here, the operation of a one-litter cylindrical centrifuge cup typically used in the United States will be discussed.

A blood bag made of soft polyvinyl chloride containing 570 ml of a whole blood preparation treated for anti-aggregation, a blood processing filter, a bag made of soft polyvinyl chloride containing about 100 ml of an erythrocyte preservation solution, an empty bag for transferring thrombocyte-rich plasma after centrifugation, and an empty bag to store the blood after processing with the blood processing filter are arranged in this centrifuge cup in the order to be centrifuged Tubes made of soft polyvinyl chloride to connect the bags to the filter are appropriately arranged between the bags and the filter. Each bag and the filter are pushed to the bottom of the centrifuge cup by centrifugal force. The bag containing the whole blood preparation and the bag containing the erythrocyte preservation solution are deformed to inflate. As a result, the flexible blood processing filter placed between the two blood bags may be crushed by the blood bags or may be deformed into a configuration conforming to the inflated blood bags. As a result, the sealed portion of the filter element and flexible container denatured into hard plastic due to welding and the like is bent, resulting in formation of cracks and peeling and giving rise to leakage. If the sealed portion is pushed to the bottom of the centrifuge cup, the sealed portion may crack due to the stress and cause leakage. If a member similar to the protruding filter material of the present invention is provided, the member acts as a cushion against the stress created by the deformation or pushing to the bottom. The distortion generated in the sealed portion of the filter element and the flexible container is reduced, thereby protecting the sealed portion. As a result, the risk of damage is remarkably decreased. At the same time, the protruding filter material prevents the adjacent blood bags and tubes in the centrifuge cup from directly contacting the sealed portion of hardened plastic, thereby protecting the blood bags and tubes from being damaged.

If the width of the protruding filter material is less than 2 mm, a sufficient effect may not be obtained. On the other hand, the width more than 25 mm lacks practical advantage, although the function and effect will not be affected. When applied to the above-described typical centrifuge cup, the width of the protruding filter material not contributing to the filtration occupies about two-thirds of the whole width of the filter. Although a width of 2 mm or more is sufficient for the protruding filter material, 3 mm or more is preferable in view of mass production and ease of handling, with 4 mm or more being more preferable, and 5 mm or more being most preferable. Although a width of 25 mm or less is practicable for the protruding filter material, 20 mm or less is preferable in view of the duration of leak inspection, with 15 mm or less being more preferable, and 10 mm or less being most preferable.

It is desirable that the width of the protruding filter material be uniform, with the difference between the largest width and the smallest width being 3 mm or less, more preferably 2 mm or less, and still more preferably 1 mm or less. A large difference between the largest width and the smallest width may result in a risk of concentrating the stress on the smallest width portion during centrifugal operation. Therefore, too large a width difference is undesirable.

The protruding filter material may be formed from all components forming the filter element in the effective filtration area or may be formed from a part of such components. The components may be appropriately selected to the extent that the desired cushion effect can be obtained. However, using all components forming the filter element in the effective filtration area is more preferable in view of simplicity of the manufacturing process.

It is necessary that the second seal zone be formed over the entire circumference at the outside of the first seal zone, and the inlet port side flexible container be integrated with the outlet port side flexible container. Due to this configuration the filter can avoid the risk of exposing the medical workers to the danger of infection or prevent the blood preparations from being contaminated with miscellaneous bacteria, even if leakage is brought by breakage of the first seal zone during the filtering operation due to operational mistakes or rough handling, stress of centrifugal operation, or the like.

The second seal zone can be formed by joining a flexible container with another flexible container. Although known methods such as internal welding by high frequency welding and supersonic wave welding, external welding by heat sealing, and adhesion using a solvent can be applied, high frequency welding is preferably used when the flexible container is made of a material with a comparatively high dielectric constant, and heat sealing is preferably used when the material has a low dielectric constant and a low melting point.

The width of the second seal zone is preferably 1-10 mm, and more preferably 2-5 mm. If less than 1 mm, satisfactorily sealing performance may not be relied upon. A width not exceeding 10 mm is desirable because an unnecessarily wide weld increases loss of raw materials.

The flexible container of the present invention may be formed from either a sheet of film or a cylindrical film. When the blood processing filter is formed from a sheet of film, the filter element may be inserted between two sheets of film or within a sheet of folded film. When the first seal zone is formed by inserting the filter element within a sheet of folded film, the second object of the present invention can be achieved by sealing only the open three sides without forming a second seal zone over the entire circumference. This feature is also within the scope of the present invention. When the first seal zone is formed by placing the first filter element inside a cylindrical film, the second object of the present invention can be achieved by sealing only the open two sides without forming a second seal zone over the entire circumference. This feature is also within the scope of the present invention.

It is essential that an unsealed zone, surrounded by the flexible container, the first seal zone, and the second seal zone, be formed between the first seal zone and the second seal zone. The width of the unsealed zone should be within a range of 1-30 mm. When the width is less than 1 mm, the filter element may be engaged with the area when the second seal zone is caused to adhere. In addition, it is difficult to detect leakage in the first seal zone as will be discussed hereinafter. A width exceeding 30 mm, on the other hand, is not practical because it takes a long time to detect leakage in the first seal zone.

Leakage in the first seal zone can be detected by a method of inspection comprising, for example, closing the tube on the outlet side of the blood processing filter with a clamp, feeding air under a pressure of 0.02 MPa from the inlet side, and maintaining this condition for a prescribed period of time, for example, for 1 minute to 1 hour, according to the width of the unsealed zone. In this instance, if leakage occurs, the clearance between the welded portions of the circumference (the part indicated by h in the drawings) is inflated. Leakage can be inspected by observing the inflation.

In the filter of the present invention, since a clearance of 1-30 mm is provided between the first seal zone and the second seal zone, the inner pressure of the filter decreases when leakage occurs in the first seal zone. Consequently, the leakage can be detected by measuring the pressure change, or by visually observing the deformation of the unsealed zone due to swelling resulting from the pressure of air coming into the unsealed zone by leakage. Leakage can be easily detected in this manner. The width of the unsealed zone is preferably 2 mm or more, and more preferably 4 mm or more, taking into account the reliability and ease of leak inspection. From the viewpoint of efficiency of leak inspection, the width of the unsealed zone is preferably 20 mm or less, and more preferably 10 mm or less.

Leakage cannot be detected by this inspection method when using the filter disclosed in WO 95/17236, in which the seal zone at the edge of the filter element is covered by being welded with the container material. In this type of filter, even when leakage causing bypassing of the blood occurs in the seal zone at the edge of the filter element, the inner pressure of the filter does not change because the blood does not leak to the outside of the filter.

In addition, when a non-woven fabric protrudes beyond the unsealed zone, the width of the unsealed zone is preferably larger by 1 mm or more, preferably by 2 mm or more, than the width of the protruding non-woven fabric. If less than 1 mm, part of the protruding filter material is engaged with the second seal zone, resulting in impaired appearance and decreased reliability of the second seal zone. On the other hand, the width of the non-seal zone need not be larger by more than 10 mm than the width of the protruding filter material. An unnecessarily wide area impairs ease of handling. A width of the non-seal zone 2-5 mm larger than the protruding filter material is more preferable, with a width 3-4 mm larger being ideal. As described above, a cushion effect can be obtained if the protruding filter material is arranged in the area.

An embodiment and a manufacturing process of the blood processing filter of the present invention are shown in the attached drawing, which should not be construed as limiting the present invention.

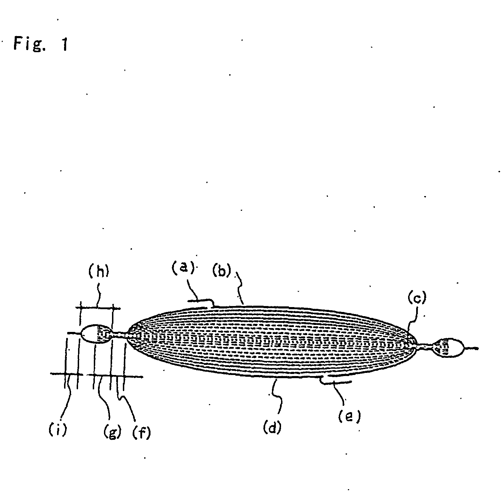

Figure 1 is a cross-sectional view of the blood processing filter comprising an inlet port side flexible container made of a resin sheet (b) equipped with a blood inlet port (a), an outlet port side flexible container made of a resin sheet (d) equipped with a blood outlet port (e), and a filter element (c) for removing undesirable components from blood, wherein the blood inlet port (a) and outlet port (e) are separated by the filter element (c) . The filter element (c) is inserted between the inlet port side flexible container and outlet port side flexible container and the vicinity of the periphery is integrated with the flexible container over the entire circumference. A second seal zone (i), integrated by welding the inlet port side flexible container and the outlet port side flexible container, is formed outside the integrated first seal zone (f). An unsealed zone (h) surrounded by the inlet port side flexible container, the outlet port side flexible container, the first seal zone (f), and the second seal zone (i) is formed between the first seal zone (f) and the second seal zone (i). When the first seal zone (f) is formed slightly inside the outermost periphery of the filter element (c), a non-sealed filter element (g) is provided protruding from the first seal zone (f).

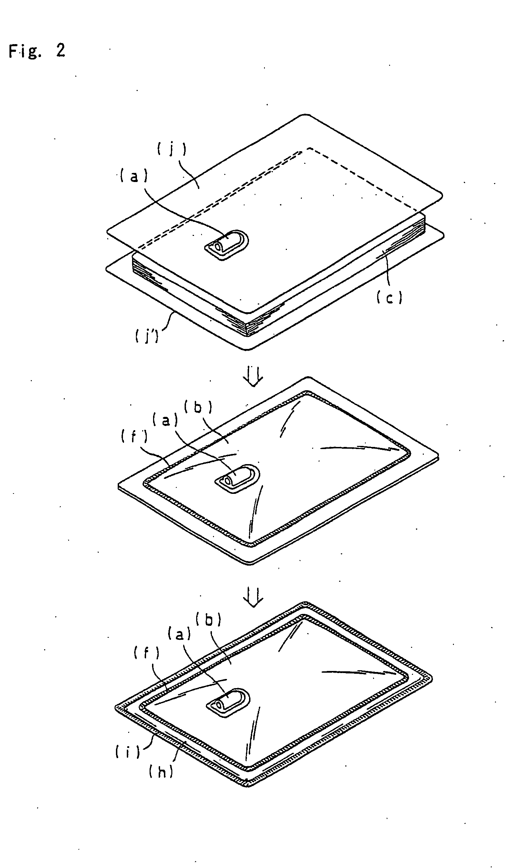

Figure 2 shows one embodiment of the process for manufacturing the blood processing filter of the present invention. A filter element (c) is inserted between two sheets of film (j) , (j'), having either an inlet port (a) or an outlet port, and a first seal zone (f) is formed by heat sealing. A second seal zone (i) is further formed to provide an unsealed zone(h).

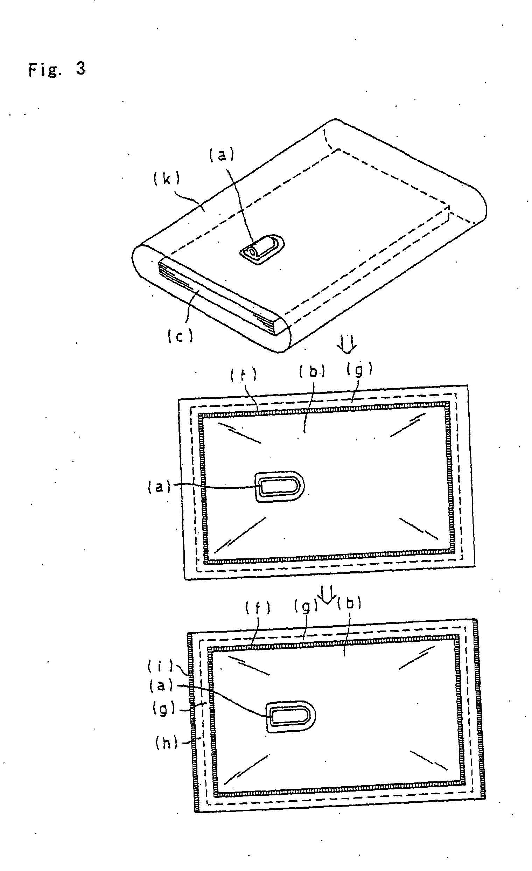

Figure 3 shows another embodiment of the process for manufacturing the blood processing filter of the present invention, wherein the flexible container is formed from a cylindrical film. A filter element (c) is inserted between a cylindrical film (k) with an inlet (a) and an outlet being formed therein, and a first seal zone (f) is formed by heat sealing. A second seal zone (i) is further formed to provide an unsealed zone(h). In this case, the second seal zone (i) may be formed only on the open end.

### EXAMPLES

The leukocyte removing filter of the present invention will now be described in detail by way of examples, which should not be construed as limiting the present invention. The following leak inspection method was used in Examples and Comparative Examples.

### (Measuring method)

### (1) Sterilization and centrifugation

An integral system consisting of a blood filter of the present invention, a blood collecting bag A, a bag B for transferring thrombocyte-rich plasma or blood plasma after centrifugation, a bag C containing about 100 ml of erythrocyte preservation solution, a bag D for receiving blood components treated by the blood processing filter after centrifugation, and tubes connecting these parts was prepared. A tube 1 for collecting blood was connected to the upper part of the bag A. Next, another tube 2, of which the one end was connected to the upper part of the bag A, was branched via a Y-branched tube and the other ends were connected with the bag B and bag C. A third tube 3 extending from the top of the bag A to the bag D was provided. The blood processing filter was joined at about middle of the tube 3. After sterilizing the system with high pressure vapor (at 121°C for 20 minutes) , the bag A was charged with 570 ml of bovine whole blood containing CPD (citrate-phosphate-dextrose) through the tube 1. The tube 1 was sealed by heat sealing at about 10 cm from the bag A and the other end was cut and separated. The system was placed in a cylindrical centrifugal cup with an internal capacity of about 1 1 in the order of the bag A, blood processing filter, bag C, bag D, and bag B. The tubes were appropriately inserted in the void spaces of the bags or the blood processing filter. The system was centrifuged using the following device and under the following conditions.
Centrifuge device: CR7B3 (manufactured by Hitachi, Ltd.)
Radius of rotation: 0.261 m
Rotational speed: 4140 rpm
Centrifuge duration: 10 minutes
Dimension of cup: internal diameter 100 mm, height: 150 mm

### (2) Leak inspection method of a blood processing filter with only the first seal zone sealed

Tubes are connected to the blood inlet port and the blood outlet port, respectively. The outlet port side tube is closed with a clamp, and air is injected from the blood inlet port side tube at a pressure of 0.02 MPa. The blood filter is kept under the water surface for several minutes. Leakage is judged by generation of air bubbles (hereinafter referred to as "underwater leak inspection").

### (3) Leak inspection method of a blood processing filter in which the first and second seal zones are sealed

Tubes are connected to the blood inlet port side and the blood outlet port side, respectively. The outlet side tube is closed with clamp, and air is injected from the blood inlet port side tube at a pressure of 0.02 MPa. The blood filter was kept in air for a time. If leakage occurs, the unsealed zone (h) surrounded by the first and second seal zones is inflated. Leakage can be inspected by observing this part. The time when the inflation of the unsealed zone was confirmed by visual inspection was measured for leaking filters (hereinafter referred to as "visual inspection").

### Example 1

Flexible polyvinyl chloride resin sheets (b, d), which were cut to a size 20 mm larger than the size of the blood filter and in which holes were made at the portions corresponding to the blood inlet port and outlet port, and a blood inlet port (a) and a blood outlet port (e), formed from a polyvinyl chloride resin by injection molding, were bonded by high frequency welding, to produce an inlet port side flexible container (b) provided with the blood inlet port (a) and an outlet port side flexible container (d) provided with the blood outlet port (e) .

The polyester non-woven fabric described below was laminated for use as the filter element (c). Four sheets of non-woven fabric (non-woven fabric 1) with a mean fiber diameter of 12-15 µm and nicking (Metsuke) of 29-31 g/m² were laminated for use as the first filter element. Total of 27 sheets of the non-woven fabric consisting of one sheet of non-woven fabric (non-woven fabric 2) with a mean fiber diameter of 1.5-2.0 µm and nicking of 65-67 g/m², 25 sheets of non-woven fabric (non-woven fabric 3) with a mean fiber diameter of 1.2-1.4 µm and nicking of 39-41 g/m², and one sheet of the non-woven fabric 2 were laminated in that order for use as the second filter element. Four sheets of the non-woven fabric (non-woven fabric 1) were laminated for use as the third filter element. The first, second, and third filter elements were laminated in that order. The laminated body comprising a total of 35 sheets of the non-woven fabric as prepared above was cut into a size of 85 mm x 68 mm (rectangle) for use as the filter element (c) . The flexible containers (b, d) and the filter element (c) were layered in the order of the inlet port side flexible container (b), filter element (c), and outlet port side flexible container (d) and welded by high frequency welding to form a filtering section with the dimension of 75 mm x 58 mm and the first seal zone with the width (f) of the first seal zone of 3 mm. The width of the protruding filter element (g) was 2 mm. High frequency welding was purposely applied under a non-optimal condition for forming the first seal zone so that leakage would be occurred with a certain probability. The resulting blood filters in which only the first seal zone has been sealed were inspected according to the underwater inspection method, and the filters were classified into leaky filters and non-leaky filters.

The protruding filter element (g) is impregnated with water and water drops adhere to the surfaces of the sheets to serve as the unsealed zone (h) and the second seal zone (i) after the underwater leak inspection. Therefore, these filters were once dried, and the flexible containers (b, d) were welded with the filters by high frequency welding so that the width of the unsealed zone (h) was 6 mm and the width of the second seal zone (i) was 3 mm. The final shape as shown in Figure 1 was obtained by cutting the outermost periphery. Ten of final shaped leaky filters and ten of non-leaky filters were inspected by visual inspection. The results are shown in Table 1.

### Example 2

A filter was prepared in the same manner as in Example 1 except that the first seal zone was welded using a sheet with a size of 82 mm x 65 mm prepared by cutting the filter element (c) so that the protruding filter element (g) with a width of 0.5 mm was provided and, after the leak inspection of the first seal zone, the flexible containers (b, d) were welded so that the unsealed zone (h) with a width of 1 mm is provided. The leak inspection was carried out according to the method described above. The results are shown in Table 1.

### Example 3

A filter was prepared in the same manner as in Example 1 except that the first seal zone was welded using a sheet with a size of 83 mm x 66 mm prepared by cutting the filter element (c) so that the protruding filter element (g) is provided with a width of 1 mm and, after the leak inspection of the first seal zone, the flexible containers (b, d) were welded so that the unsealed zone (h) is provided with a width of 2 mm. The leak inspection was carried out according to the method described above. The results are shown in Table 1.

### Example 4

A filter was prepared in the same manner as in Example 1 except that the flexible containers (b, d) were welded so that the unsealed zone (h) of the blood filter is provided with a width of 4 mm. Leakage was inspected by the method described above. The results are shown in Table 1.

### Example 5

A filter was prepared in the same manner as in Example 1 except that the flexible containers (b, d) were welded so that the unsealed zone (h) of the blood filter is provided with a width of 10 mm. Leakage was inspected by the method described above. The results are shown in Table 1.

### Example 6

A filter was prepared in the same manner as in Example 1 except that the flexible containers (b, d) were welded so that the unsealed zone (h) of the blood filter is provided with a width of 20 mm. Leakage was inspected by the method described above. The results are shown in Table 1.

### Example 7

A filter was prepared in the same manner as in Example 1 except that the flexible containers (b, d) were welded so that the sealed zone (h) of the blood filter is provided with a width of 30 mm. Leakage was inspected by the method described above. The results are shown in Table 1.

**TABLE 1**

| | | Example | | | | | | |
|---|---|---|---|---|---|---|---|---|
| | | 1 | 2 | 3 | 4 | 5 | 6 | 7 |
| Filter size (mm) | length | 99 | 89 | 91 | 95 | 107 | 127 | 147 |
| | width | 82 | 72 | 74 | 78 | 90 | 110 | 130 |
| Size of filter element outermost circumference (mm) | length | 85 | 82 | 83 | 85 | 85 | 85 | 85 |
| | width | 68 | 65 | 66 | 68 | 68 | 68 | 68 |
| Size of Blood filter filtration area (mm) | length | 75 | 75 | 75 | 75 | 75 | 75 | 75 |
| | width | 58 | 58 | 58 | 58 | 58 | 58 | 58 |
| Width of first seal zone f (mm) | | 3 | 3 | 3 | 3 | 3 | 3 | 3 |
| Width of protruding seal zone g (mm) | | 2 | 0.5 | 1 | 2 | 2 | 2 | 2 |
| Width of non-seal zone h (mm) | | 6 | 1 | 2 | 4 | 10 | 20 | 30 |
| Width of second seal zone i (mm) | | 3 | 3 | 3 | 3 | 3 | 3 | 3 |
| Leak inspection ratio for fist seal zone leak product | | 10/10 | 10/10 | 10/10 | 10/10 | 10/10 | 10/10 | 10/10 |
| Leak inspection ratio for fist seal zone non-leak product | | 0/10 | 0/10 | 0/10 | 0/10 | 0/10 | 0/10 | 0/10 |
| Leak inspection time for fist seal zone leak product (sec) | | 13 13 | 2 2 | 4 4 | 8 | 60 60 | 600 600 | 1800 180 |

### Comparative Example 1

A filter was prepared in the same manner as in Example 1 except that the first seal zone was welded using a sheet with a size of 81 mm x 64 mm prepared by cutting the filter element (c) so that the protruding filter element (g) is provided with a width of 0 mm and, after the leak inspection of the first seal zone, the flexible.containers (b, d) were welded so that the unsealed zone (h) is provided with a width of 0 mm. It was difficult for the first seal zone to be welded in a stable manner by high frequency welding due to frequent sparks. In addition, in some cases the end of the first seal zone invaded the second seal zone, making it impossible to weld the second seal zone. The filters without these problems were selected for leak inspection. However, the visual inspection could not judge whether or not these filters were leaky or non-leaky. The results of the visual inspection are shown in Table 2.

### Comparative Example 2

A filter was prepared in the same manner as in Example 2 except that the flexible containers (b, d) were welded so that the unsealed zone (h) of the blood filter is provided with a width of 0.5 mm. In some cases, the protruding filter element (g) invaded the second seal zone, making it impossible to weld the second seal zone. The filters without these problems were selected for leak inspection. However, it was difficult to judge whether or not these filters were leaky or non-leaky by visual inspection. The results of the visual inspection are shown in Table 2.

### Comparative Example 3

A filter was prepared in the same manner as in Comparative Example 1 except that the flexible containers (b, d) were welded so that the unsealed zone (h) of the blood filter is provided with a width of 0.5 mm. Stable welding was difficult as in the case of Comparative Example 1, and it was difficult to judge whether or not these filters were leaky or non-leaky by visual inspection as in the case of Comparative Example 2. The results of the visual inspection are shown in Table 2.

### Comparative Example 4

A filter was prepared in the same manner as in Example 0 except that the first seal zone was welded using a sheet with a size of 81.6 mm x 64.6 mm prepared by cutting the filter element (c) so that the protruding filter element (g) with a width of 0.3 mm was provided and, after the leak inspection of the first seal zone, the flexible containers (b, d) were welded so that the unsealed zone (h) with a width of 0.5 mm is provided. The leak inspection was carried out according to the method described above. The results are shown in Table 2.

### Comparative Example 5

A filter was prepared in the same manner as in Example 1 except that the flexible containers (b, d) were welded so that the unsealed zone (h) of the blood filter is provided with a width of 35 mm. Leakage was inspected by the method described above. The results are shown in Table 2.

**TABLE 2**

| | | Example | | | | |
|---|---|---|---|---|---|---|
| | | 1 | 2 | 3 | 4 | 5 |
| Filter size (mm) | length | 87 | 88 | 88 | 88 | 157 |
| | width | 70 | 71 | 71 | 71 | 140 |
| Size of filter element outermost circumference (mm) | length | 81 | 82 | 81 | 81.6 | 85 |
| | width | 64 | 65 | 64 | 64.6 | 68 |
| Size of Blood filter filtration area (mm) | length | 75 | 75 | 75 | 75 | 75 |
| | width | 58 | 58 | 58 | 58 | 58 |
| Width of first seal zone f (mm) | | 3 | 3 | 3 | 3 | 3 |
| Width of protruding seal zone g (mm) | | 0 | 0.5 | 0 | 0.3 | 2 |
| Width of non-seal zone h (mm) | | 0 | 0.5 | 0.5 | 0.5 | 35 |
| Width of second seal zone i (mm) | | 3 | 3 | 3 | 3 | 3 |
| Leak inspection ratio for fist seal zone leak product | | 0/10 | 2/10 | 5/10 | 3/10 | 10/10 |
| Leak inspection ratio for fist seal zone non-leak product | | 0/10 | 0/10 | 0/10 | 0/10 | 0/10 |
| Leak inspection time for fist seal zone leak product (sec) | | - | 20 | 20 | 20 | 3600 |

The results of Tables 1 and 2 indicate that if the unsealed zone has a width of 1-30 mm, superior results are obtained in both the leakage ratio and the time required for inspection.

### Example 8

The inlet port side flexible container (b) , outlet port side flexible container (d), and the filter element (c) were prepared in the same manner as in Example 1.

The flexible containers (b, d) and the filter element (c) were layered as shown in Figure 1 andweldedby high frequency one step welding to form a filtering area with a dimension of 75 x 58 mm and the first seal zone with a width (f) of 3 mm. The width (g) of the protruding filter element was 2 mm, with the difference between the maximum width and minimum width being 1 mm or less. Flexible containers (b, d) were prepared by high frequency welding so that the unsealed zone is provided with a width (h) of 5 mm and the second seal zone is provided with a width (i) of 3 mm. The outermost periphery was cut to obtain the final shape shown in Figure 1. The blood processing filters were inspected by the method described above. The filters exhibiting no leakage in the first seal zone were used for the above-described sterilization/centrifuge operation. The leakage was inspected according to the above-described method. The results are shown in Table 3.

### Example 9

A blood processing filter was prepared in the same manner as in Example 8, except that a filter material cut to a size of 87 x 70 mm was used to prepare a protruding filter element with a width (g) of 3 mm, and the unsealed zone was provided with a width of 6 mm. The difference between the maximum width and minimum width of the protruding filter element was 1 mm. The filters were used for the above-described sterilization/centrifuge operation. The leakage was inspected according to the above-described method before and after the sterilization/centrifuge operation. The results are shown in Table 3.

### Example 10

A blood processing filter was prepared in the same manner as in Example 8, except that a filter material cut to a size of 89 x 72 mm was used to prepare a protruding filter element with a width (g) of 4 mm, and the unsealed zone was provided with a width of 7 mm. The difference between the maximum width and minimum width of the protruding filter element was 1 mm. The leakage was inspected according to the above-described method before and after the sterilization/centrifuge operation. The results are shown in Table 3.

### Example 11

A blood processing filter was prepared in the same manner as in Example 8, except that a filter material cut to a size of 91 x 74 mm was used to prepare a protruding filter element with a width (g) of 5 mm, and the unsealed zone was provided with a width of 8 mm. The difference between the maximum width and minimum width of the protruding filter element was 1 mm. The leakage was inspected according to the above-described method before and after the sterilization/centrifuge operation. The results are shown in Table 3.

### Example 12

A blood processing filter was prepared in the same manner as in Example 8, except that a filter material cut to a size of 97 x 80 mm was used to prepare a filtering area with a size of 71 x 54 mm, a protruding filter element with a width (g) of 10 mm, and the unsealed zone was provided with a width of 13 mm. The difference between the maximum width and minimum width of the protruding filter element was 1 mm. The leakage was inspected according to the above-described method before and after the sterilization/centrifuge operation. The results are shown in Table 3.

### Example 13

A blood processing filter was prepared in the same manner as in Example 8, except that a filter material cut to a size of 87 x 80 mm was used to prepare a filtering area with a size of 51 x 44 mm, a protruding filter element with a width (g) of 15 mm, and the unsealed zone was provided with a width of 18 mm. The difference between the maximum width and minimum width of the protruding filter element was 1 mm. The leakage was inspected according to the above-described method before and after the sterilization/centrifuge operation. The results are shown in Table 3.

### Example 14

A blood processing filter was prepared in the same manner as in Example 8, except that a filter material cut to a size of 87 x 80 mm was used to prepare a filtering area with a size of 31 x 24 mm, a protruding filter element with a width (g) of 25 mm, and the unsealed zone was provided with a width of 28 mm. The difference between the maximum width and minimum width of the protruding filter element was 1 mm. The leakage was inspected according to the above-described method before and after the sterilization/centrifuge operation. The results are shown in Table 3.

### Example 15

A blood processing filter was prepared in the same manner as in Example 11, except that a filter material cut to a size of 93 x 76 mm to prepare a first seal zone with a width (f) of 4 mm was used. The difference between the maximum width and minimum width of the protruding filter element was 1 mm. The leakage was inspected according to the above-described method before and after the sterilization/centrifuge operation. The results are shown in Table 3.

### Example 16

A blood processing filter was prepared in the same manner as in Example 11, except that a filter material cut to a size of 97 x 80 mm to prepare a first seal zone with a width (f) of 6 mm was used. The difference between the maximum width and minimum width of the protruding filter element was 1 mm. The leakage was inspected according to the above-described method before and after the sterilization/centrifuge operation. The results are shown in Table 3.

### Example 17

A blood processing filter was prepared in the same manner as in Example 8, except that a filter material cut to a size of 83 x 66 mm was used to prepare a first seal zone with a width (f) of 2 mm and a protruding filter element with a width (g) of 2 mm. The difference between the maximum width and minimum width of the protruding filter element was 1 mm. The leakage was inspected according to the above-described method before and after the sterilization/centrifuge operation. The results are shown in Table 3.

### Example 18

A blood processing filter was prepared in the same manner as in Example 17, except that a filter material cut to a size of 93 x 76 mm was used to prepare a first seal zone with a width (f) of 2 mm and a protruding filter element with a width (g) of 7 mm, and the unsealed zone was provided with a width of 10 mm. The difference between the maximum width and minimum width of the protruding filter element was 1 mm. The leakage was inspected according to the above-described method before and after the sterilization/centrifuge operation. The results are shown in Table 3.

### Example 19

A blood processing filter was prepared in the same manner as in Example 8, except that a filter material cut to a size of 97 x 80 mm was used to prepare a filtering area with a size of 63 x 46 mm, a first seal zone with a width (f) of 7 mm, and a protruding filter element with a width (g) of 10 mm, and the unsealed zone was provided with a width of 13 mm. The difference between the maximum width and minimum width of the protruding filter element was 1 mm. The leakage was inspected according to the above-described method before and after the sterilization/centrifuge operation. The results are shown in Table 3.

### Example 20

A blood processing filter was prepared in the same manner as in Example 19, except that a filter material cut to a size of 87 x 80 mm was used to prepare a filtering area with a size of 43 x 36 mm, a first seal zone with a width (f) of 7 mm, and a protruding filter element with a width (g) of 15 mm, and the unsealed zone was provided with a width of 18 mm. The difference between the maximum width and minimum width of the protruding filter element was 1 mm. The leakage was inspected according to the above-described method before and after the sterilization/centrifuge operation. The results are shown in Table 3.

### Example 21

The filters were prepared in the same manner as in Example 11 and those having a difference between the maximum width and minimum width of the protruding filter element of 2 mm were subjected to the leak inspection according to the above-described method before and after the sterilization/centrifuge operation. The results are shown in Table 3.

### Example 22

The filters were prepared in the same manner as in Example 11 and those having a difference between the maximum width and minimum width of the protruding filter element of 3 mm were subjected to the leak inspection according to the above-described method before and after the sterilization/centrifuge operation. The results are shown in Table 3.

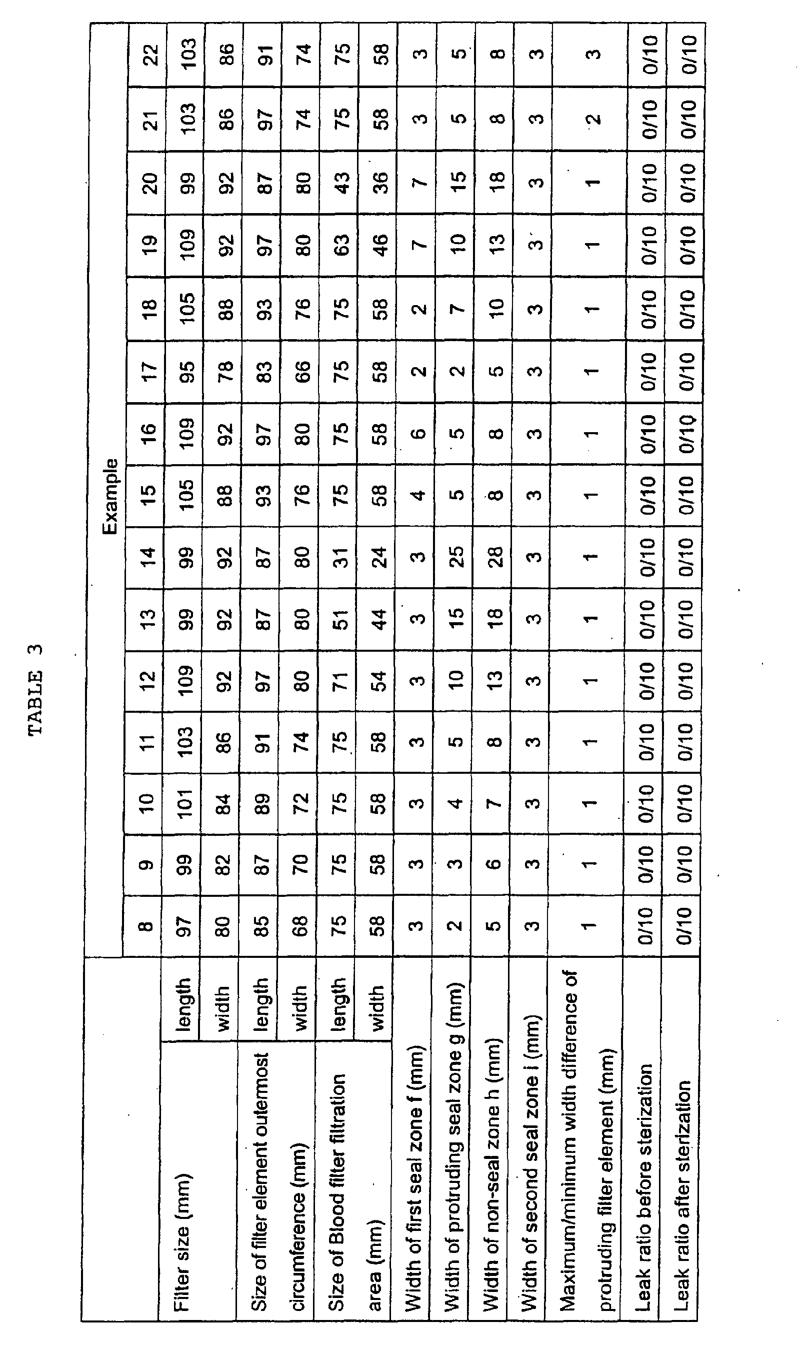

### Comparative Example 6

A blood processing filter was prepared in the same manner as in Example 8, except that a filter material cut to a size of 83 x 66 mm was used to prepare a protruding filter element with a width (g) of 1 mm, and the unsealed zone was provided with a width of 4 mm. The difference between the maximum width and minimum width of the protruding filter element was 0.5 mm. The leakage was inspected according to the above-described method before and after the sterilization/centrifuge operation. The results are shown in Table 4.

### Comparative Example 7

A blood processing filter was prepared in the same manner as in Example 8, except that a filter material cut to a size of 87 x 80 mm was used to prepare a filtering area with a size of 25 x 18 mm, a protruding filter element with a width (g) of 28 mm, and the unsealed zone was provided with a width of 31 mm. The difference between the maximum width and minimum width of the protruding filter element was 1 mm. The leakage was inspected according to the above-described method before and after the sterilization/centrifuge operation. The results are shown in Table 4.

**TABLE 4**

| | | Comparative Example | |
|---|---|---|---|
| | | 6 | 7 |
| Filter size (mm) | length | 95 | 99 |
| | width | 78 | 92 |
| Size of filter element outermost circumference (mm) | length | 83 | 87 |
| | width | 66 | 80 |
| Size of Blood filter filtration area (mm) | length | 75 | 25 |
| | width | 58 | 18 |
| Width of first seal zone f (mm) | | 3 | 3 |
| Width of protruding seal zone g (mm) | | 1 | 28 |
| Width of non-seal zone h (mm) | | 4 | 31 |
| Width of second seal zone i (mm) | | 3 | 3 |
| Maximum/minimum width difference of protruding filter element (mm) | | 0.5 | 1 |
| Leak ratio before sterization | | 0/10 | 0/10 |
| Leak ratio after sterization | | 4/10 | 0/10 |

The results of Tables 3 and 4 indicate that if the protruding filter element is provided with a width of 2-25 mm and the width variation is as small as 3 mm or less, in terms of the difference between the maximum width and the minimum width, the filter exhibits only a small leak ratio after the centrifuge operation and the welded portion is not broken by a stress during the centrifuge operation.

### INDUSTRIAL APPLICABILITY

A flexible blood processing filter can be manufactured without using a sheet-like flexible frame according to the present invention. The flexible blood processing filter can protect the medical workers from the risk of exposure to infections or prevent contamination of the blood preparations with bacteria, and can inspect and detect the risk of cracks and the like which may result in decreased leukocyte removing function.

In addition, a blood processing filter which is free from the risk of decreasing the leukocyte removing function due to breakage of welding portions by a stress during centrifugal operation can be provided according to the present invention.

## Claims

1. In a blood processing filter comprising a flexible container having an inlet port and an outlet port for the blood and a sheet-like filter element for removing undesirable components from blood, wherein the filter element separates the inlet port from the outlet port for the blood, a blood processing filter **characterized by** comprising: a first seal zone formed by integrating the entire circumference in the vicinity of the periphery of the filter element with the flexible container; a second seal zone formed by integrating the inlet port side flexible container with the outlet port side flexible container over the entire outer circumference of the first seal zone; and an unsealed zone with a width of 1-30 mm between the first seal zone and the second seal zone.

2. In a blood processing filter comprising a flexible container having an inlet port and an outlet port for the blood and a sheet-like filter element for removing undesirable components from blood, wherein the filter element separates the inlet port from the outlet port for the blood, a blood processing filter **characterized by** comprising: a first seal zone formed by integrating the entire circumference inside the periphery of the sheet-like filter element with the flexible container; a second seal zone formed by integrating the inlet port side flexible container with the outlet port side flexible container over the entire outer circumference of the first seal zone; an unsealed zone between the first seal zone and the second seal zone; and a peripheral end of the filter element with a width of 2-25 mm over the entire circumference of the filter element within the unsealed zone.

3. The blood processing filter according to claim 2, wherein the width of the peripheral end of the filter element within the unsealed zone has a variation as small as 3 mm or less, in terms of the difference between the maximum width and the minimum width.

4. The blood processing filter according to any one of claims 1-3, wherein the sheet-like filter element comprises at least a filter element for removing leukocytes.

5. The blood processing filter according to any one of claims 1-4, wherein the filter element comprises a first filter element for removing aggregates from blood, a second filter element arranged downstream of the first filter element to remove leukocytes, and a third filter element arranged between the second filter element and the outlet port side container to prevent adhesion of the second filter element to the outlet port side container.

6. The blood processing filter according to claim 4 or 5, wherein the first seal zone is integrally formed with the flexible container at least in the entire circumference of the vicinity of the periphery of the filter element for removing leukocytes.

7. The blood processing filter according to any one of claims 1-6, wherein the flexible container is formed from a sheet-like formed material.

8. The blood processing filter according to any one of claims 1-6, wherein the flexible container is formed from a cylindrical formed material.

9. The blood processing filter according to any one of claims 1-8, wherein the inlet port and outlet port made of a formed material are liquid-tightly joined with the flexible container.

10. The blood processing filter according to any one of claims 1-9, wherein the flexible container is formed from soft polyvinyl chloride.

11. The blood processing filter according to any one of claims 1-9, wherein the flexible container is formed from polyolefin.
